# EUROPÄISCHE PATENTSCHRIFT

(11) **EP 2 914 205 B1**
(45) Veröffentlichungstag und Bekanntmachung des Hinweises auf die Patenterteilung: **04.01.2017**
(21) Anmeldenummer: 13798261.7
(22) Anmeldetag: 04.11.2013
(51) Int. Cl.: A61C 19/04, G01B 11/25, A61B 5/107, G06T 7/00, G01B 21/04

(54) **KALIBRIERUNGSVORRICHTUNG UND VERFAHREN ZUR KALIBRIERUNG EINER DENTALEN KAMERA**
CALIBRATION DEVICE AND METHOD FOR CALIBRATING A DENTAL CAMERA
DISPOSITIF D'ÉTALONNAGE ET PROCÉDÉ D'ÉTALONNAGE D'UNE CAMÉRA DENTAIRE

(30) Priorität: 02.11.2012 DE 102012220048
(43) Veröffentlichungstag der Anmeldung: 09.09.2015
(73) Patentinhaber: Sirona Dental Systems GmbH, 64625 Bensheim (DE)
(72) Erfinder: POPILKA, Björn, 69502 Hemsbach (DE); THIEL, Frank, 64372 Ober-Ramstadt (DE); FORSTER, Frank, 81739 München (DE)
(74) Vertreter: Sommer, Peter
(86) Internationale Anmeldenummer: PCT/EP2013/072884
(87) Internationale Veröffentlichungsnummer: WO 2014/068097

(56) Entgegenhaltungen:
- EP-A1- 1 650 529
- EP-A1- 1 757 902
- WO-A1-2006/036110
- DE-A1- 10 133 568
- DE-A1- 19 536 297
- US-A- 5 991 437
- US-A1- 2002 006 217
- US-A1- 2012 092 461

## Beschreibung

### Technisches Gebiet

Die Erfindung betrifft eine Kalibrierungsvorrichtung und ein Verfahren zur Kalibrierung einer dentalen Kamera, die auf einem Streifenprojektionsverfahren zur dreidimensionalen optischen Vermessung eines dentalen Objekts beruht, umfassend ein Projektionsgitter zur Erzeugung eines Projektionsmusters aus mehreren Streifen und ein optisches System, das das erzeugte Projektionsmuster auf das zu vermessende Objekt projiziert.

### Stand der Technik

Aus dem Stand der Technik sind mehrere Streifenprojektionsverfahren zur dreidimensionalen optischen Vermessung bekannt. Dabei wird ein Projektionsmuster auf das zu vermessende Objekt projiziert. Anschließend werden ausgehend vom verzerrten Projektionsmuster unter Verwendung eines Triangulationsverfahrens 3D-Koordinaten der Messpunkte auf dem Objekt berechnet. Aus einer Wolke von Messpunkten wird dann ein 3D-Modell des Objekts berechnet.

Ein Nachteil dieses Verfahrens ist, dass Fertigungsfehler des Projektionsgitters oder optische Fehler des optischen Systems dazu führen können, dass das erzeugte 3D-Modell im Vergleich zu den tatsächlichen Abmessungen des Objekts Messfehler aufweist. Die Fertigungsfehler des Projektionsgitters können beispielsweise zu einer fehlerhaften Streifenbreite oder zu fehlerhaften Abständen zwischen den Streifen führen. Optische Fehler des optischen Systems können beispielweise Verzeichnungen sein, die das Projektionsmuster verzerren.

US 2002/006217 A1 offenbart ein Verfahren und ein System zur Kalibrierung einer Kamera, wobei eine Kalibrierungsstation genannt wird, um die Kamera zu kalibrieren. Dabei wird an zwei bekannten Abständen in z-Richtung und in x- und y-Richtung kalibriert. Das Kalibrierungssystem umfasst einen Halter für die Kamera, wobei eine erste Platte in z-Richtung zwischen festgelegten Positionen z1 und z2 verschoben wird. Eine zweite Kalibrierfläche mit kleinen Öffnungen, die hinterleuchtet werden, ermöglicht die Kalibrierung in x- und y-Richtung. Die Kalibrierflächen sind dabei senkrecht zur z-Richtung angeordnet.

EP 1 757 902 A1 offenbart ein Verfahren und eine Vorrichtung zur Formerfassung eines zahntechnischen Objekts, wobei mittels einer Referenzierungskamera, die höhenverstellbar ist zur Positionierung des Objekts verwendet wird, sodass das Objekt von der Drehachse durchsetzt wird. Mittels der Referenzierungskamera werden Referenzkörper in der Nähe des Objekts erfasst. Bei der Messung erfolgt eine schrittweise Verstellung des Objekts auf eine Halterung um die Drehachse um insgesamt 360°, wobei in jeder Position mittels zweier Matrix-Kameras das Objekt aus zwei unterschiedlichen Richtungen vermessen wird, sodass anschließend ein dreidimensionales Modell des Objekts berechnet werden kann.

US 5,991,437 A offenbart ein System für eine genaue Kalibrierung einer Vermessungskamera, wobei zur Kalibrierung die Methode der kleinsten Quadrate verwendet wird.

US 2012/092461 A1 offenbart einen Scanner zur Vermessung eines dreidimensionalen Objekts mittels eines konfokalen Musterprojektionsverfahrens, wobei davor eine Kalibrierung erfolgt. Bei der Kalibrierung wird ein ebenes Schachbrettmuster eines Kalibrierungskörpers vermessen, wobei eine Differenz zwischen den simulierten und den aufgezeichneten Aufnahmen ermittelt wird. Anschließend wird eine Tabelle mit Kalibrierungswerten für alle Pixel erzeugt, um diese Differenz auszugleichen.

DE 195 36 297 A1 offenbart ein Verfahren zur geometrischen Kalibrierung von optischen 3D-Sensoren zur dreidimensionalen Vermessung von Objekten, wobei eine Kamera an einem Beleuchtungsprojektor fixiert ist, wobei zeitlich nacheinander mehrere Lichtstrukturen auf die Kalibrierungsplatte projiziert werden, die mittels der Kamera aufgenommen werden. Anschließend erfolgt die Kalibrierung der Kamera mittels eines fotogrammetrischen Standardverfahrens.

EP 1 650 529 A1 offenbart eine Anordnung und ein Verfahren zum Abtasten mehrerer Objekte mittels Triangulation, wobei Lichtstrahlen der Lichtquellen über eine Spiegelanordnung umgelenkt werden, wobei die Spiegelanordnung aus mehreren Spiegeln besteht und eine konusförmige Form aufweist. Die Spiegel haben jeweils paarweise die gleiche Neigung gegenüber der Drehachse der Spiegelanordnung, wobei die Spiegelanordnung um die Drehachse reduziert werden kann und mittels eines Gewindes in der Höhe verstellt werden kann.

WO 2006/036110 A1 offenbart eine Scanneranordnung mit einer Basiseinheit mit einem Befestigungsteil, das am Scanner angebracht ist, wobei ein scheibenförmiges Teil am Befestigungsteil angeordnet ist, wobei das scheibenförmige Teil verschiedene Neigungspositionen ermöglicht, wobei an der Oberseite des scheibenförmigen Teils mehrere Kugelteile und ein Führungsstift angeordnet sind, die als Referenzobjekte dienen. Die Höhenverstellung des scheibenförmigen Teils erfolgt nach einem teleskopischen Prinzip, wobei zwei Vorteile ineinander geschoben werden und bei bestimmten Höheneinstellungen zueinander fixiert werden können. Das Objekt wird auf dem scheibenförmigen Teil positioniert und kann in die formierten Höheneinstellungen und aus unterschiedlichen Richtungen aufgenommen werden.

DE 101 33 568 A1 offenbart ein Verfahren zur dreidimensionalen Vermessung eines Objekts, wobei das Objekt in eine Halterung eingespannt wird, das Objekt aufgenommen wird, die Aufnahme ausgewertet wird und eine Abstandsinformation relativ zur Strahlungsquelle ermittelt wird.

Die Aufgabe der vorliegenden Erfindung besteht daher darin, ein Verfahren zur Kalibrierung einer dentalen Kamera bereitzustellen, um die durch Fertigungsfehler oder optische Abbildungsfehler verursachten Messfehler zu kompensieren.

### Darstellung der Erfindung

Die Erfindung betrifft ein Verfahren zur Kalibrierung einer dentalen Kamera, die auf einem Streifenprojektionsverfahren zur dreidimensionalen optischen Vermessung eines dentalen Objekts beruht. Die dentale Kamera umfasst dabei ein Projektionsgitter zur Erzeugung eines Projektionsmusters aus mehreren Streifen und ein optisches System, das das erzeugte Projektionsmuster auf das zu vermessende Objekt projiziert. Mittels der dentalen Kamera wird unter Verwendung des Streifenprojektionsverfahrens in einem ersten Verfahrensschritt eine Referenzoberfläche mit bekannten Abmessungen vermessen. Dabei werden Ist-Koordinaten von mehreren Messpunkten auf der Referenzoberfläche ermittelt. Anschließend werden die ermittelten Ist-Koordinaten mit bekannten Soll-Koordinaten der Messpunkte auf der Referenzoberfläche mit bekannten Abmessungen verglichen. Im weiteren Verfahrensschritt werden ausgehend von den Abweichungen zwischen den Ist-Koordinaten und den Soll-Koordinaten mehrere Kompensationswerte für die einzelnen Streifen des Projektionsmusters berechnet. Diese berechneten Kompensationswerte werden dann während der Vermessung des dentalen Objekts berücksichtigt, um die Abweichungen zwischen den Ist-Koordinaten und den Soll-Koordinaten zu kompensieren.

Die ebene Referenzoberfläche an der zweiten Haltevorrichtung ist in einem Winkel zwischen 50° und 70° relativ zu einer Mittelachse des Außengewindes angeordnet.

Der Winkel kann insbesondere 65° betragen.

Durch die Drehung erfolgt eine Höhenverstellung in Richtung der Mittelachse des Außengewindes (z-Achse) bzw. der optischen Achse der Kamera. Nach einer 360°-Drehung erreicht die Referenzoberfläche wieder den gleichen Winkel relativ zur Kamera in einer unterschiedlichen Höhenposition bzw. z-Position. Außerdem sind Aufnahmen in den Zwischenstellungen 90°, 180° und 270° vorteilhaft. Durch die Schrägstellung der Referenzoberfläche und die Drehung um die optische Achse nimmt das Target dabei abwechselnd positive bzw. negative Winkelstellungen um die x- bzw. y-Achse der Kamera ein.

Dadurch kann der Winkel der Referenzoberfläche relativ zur Kamera während der Drehung innerhalb eines breiten Winkelbereichs verändert werden, so dass mehrere Zwischenstellungen vermessen werden können.

Die Aufnahmen werden mittels einer Kalibrierungsvorrichtung durchgeführt, die eine erste Haltevorrichtung für die dentale Kamera und eine zweite Haltevorrichtung für die Referenzoberfläche aufweist, die auf eine definierte Weise in mehreren Schritten relativ zur ersten Haltevorrichtung verstellbar ist.

Eine solche Kalibrierungsvorrichtung ermöglicht es, den Abstand und die Ausrichtung der dentalen Kamera relativ zur Referenzoberfläche schrittweise zu verstellen. Damit können für unterschiedliche Abstände und unterschiedliche Winkel zwischen der Referenzoberfläche und der dentalen Kamera Aufnahmen durchgeführt werden, wobei anschließend für jede der Aufnahmen Kompensationswerte für die einzelnen Streifen ermittelt werden. Die erste Haltevorrichtung und die zweite Haltevorrichtung können beispielsweise mittels einer Schraubverbindung verbunden sein.

Die Referenzoberfläche ist eine Kalibrierungsplatte, die mit mehreren Markierungen versehen ist, wobei anhand der Markierungen die genaue Lage und Ausrichtung der Kalibrierungsplatte relativ zur dentalen Kamera bestimmt wird. Anschließend werden die Abweichungen zwischen den Ist-Koordinaten und der Kalibrierungsplatte entlang einer Richtung senkrecht zur Kalibrierungsplatte ermittelt.

Das Ergebnis der Kalibrierung ist eine Matrix aus Kompensationswerten, die in einem Kompensationsverfahrensschritt während der Vermessung des Objekts auf das Projektionsmuster aus parallelen Streifen angewendet wird, um das Projektionsmuster zu korrigieren, wobei anschließend unter Verwendung des Streifenprojektionsverfahrens ausgehend vom korrigierten Projektionsmuster eine dreidimensionale Aufnahme des Objekts berechnet wird.

Bei einem Streifenprojektionsverfahren wird ein Muster aus mehreren parallelen Streifen auf das zu vermessende Objekt projiziert und anhand der Verzerrung dieser Streifen unter Verwendung eines Triangulationsverfahrens eine dreidimensionale Aufnahme des Objekts erzeugt.

Die Streifen können beispielsweise farblich kodiert sein, um eine Identifizierung zu ermöglichen. Dabei wird ein Muster aus mehreren Farbstreifen auf das Objekt projiziert. Anschließend werden die Tiefenkoordinaten für die Messpunkte ermittelt und ein 3D-Modell des Objekts erzeugt. Die Farbstreifen können anhand ihrer Farbe eindeutig identifiziert werden. Für die farbliche Kodierung der Farbstreifen können beispielsweise vier Farbstreifen beziehungsweise drei Farbübergänge verwendet werden. Die Farbstreifen können beispielsweise mittels eines Dias erzeugt werden.

Die Streifenbreite für solche Streifenprojektionsverfahren kann beispielsweise 150 µm im Messvolumen am zu vermessenden Objekt betragen.

Zur optischen Vermessung kann auch ein anderes Streifenprojektionsverfahren verwendet werden, bei dem die Kodierung der Streifen unter Verwendung unterschiedlicher Lichteigenschaften, wie Intensität, Farbe, Polarisation, Kohärenz, Phase, Kontrast, Ort oder Laufzeit, erfolgt.

Das Projektionsgitter zur Erzeugung des Projektionsmusters kann beispielsweise ein Dia sein, das durchleuchtet wird und durch welches die parallelen Streifen erzeugt werden. Die Referenzoberfläche kann beispielsweise eine ebene oder eine beliebige dreidimensionale Oberfläche, wie eine Halbkugel, sein. Ein Fertigungsfehler des Projektionsgitters oder ein Abbildungsfehler des optischen Systems führt zu den Abweichungen zwischen den Ist-Koordinaten und den Soll-Koordinaten der Messpunkte auf der Referenzoberfläche. Zur Kompensation werden Kompensationswerte für die einzelnen Streifen berechnet. Diese Kompensationswerte können in einem Speicher abgespeichert werden und später nach der Kalibrierung während der Vermessung auf das Projektionsmuster angewendet werden, bevor die Berechnung der dreidimensionalen Aufnahme anhand des korrigierten Projektionsmusters erfolgt. Dadurch werden die Abweichungen zwischen den Ist-Koordinaten und den Soll-Koordinaten kompensiert.

Die Markierungen können beispielsweise rechteckige Markierungen sein, die in festgelegten Abständen zueinander angeordnet sind. Damit können der Abstand sowie die Ausrichtung der Referenzoberfläche relativ zur Kamera anhand der Abstände zwischen Markierungen mittels eines Computers berechnet werden. Damit ist also kein Fitten durch die Ist-Koordinaten der Messpunkte notwendig, da die Lage bereits anhand der Markierungen ermittelt wird. Abweichungen zwischen Ist-Koordinaten und der Kalibrierungsplatte können damit unmittelbar mittels eines Computers berechnet werden.

Zur Ermittlung der Lage der Referenzfläche können vorteilhafterweise die in der Literatur bekannten Kalibrierverfahren von Tsai ("A Versatile Camera Calibration Technique for High-Accuracy 3D Vision Metrology Using Off-the-Shelf Cameras and Lenses", 1987) oder Zhang ("A flexible new technique for camera calibration", 2000) verwendet werden.

Ein Vorteil dieses Verfahrens besteht darin, dass die durch Fertigungsfehler oder durch Abbildungsfehler des optischen Systems entstandenen Abweichungen kompensiert werden und dadurch die Aufnahmequalität verbessert wird.

Die Kompensationswerte einer solchen Matrix können beispielsweise Verschiebungsvektoren sein, die eine notwendige Verschiebung der jeweiligen parallelen Streifen darstellen, um die Abweichungen zwischen den Ist-Koordinaten und den Soll-Koordinaten für die jeweiligen Streifen zu kompensieren. Bei der Berechnung einer dreidimensionalen Aufnahme wird also die Matrix aus Kompensationswerten zuerst auf das Projektionsmuster angewendet und erst anschließend werden anhand des korrigierten Projektionsmusters die 3D-Koordinaten der Messpunkte auf dem Objekt berechnet.

Vorteilhafterweise kann die Referenzoberfläche eine Ebene sein und die Abweichungen zwischen den Ist-Koordinaten und den Soll-Koordinaten können ermittelt werden, indem durch die Ist-Koordinaten unter Verwendung der Methode der kleinsten Quadrate eine Bezugsebene gefittet wird und anschließend die Abweichungen zwischen den Ist-Koordinaten und der Bezugsebene entlang einer Richtung senkrecht zur Bezugsebene ermittelt werden.

Die genaue Lage der Referenzoberfläche relativ zur dentalen Kamera wird also durch das Fitten ermittelt. Bei einem fehlerfreien Projektionsgitter und bei einem fehlerfreien optischen System würden also alle Soll-Koordinaten in der Ebene der Referenzoberfläche liegen, so dass die Ist-Koordinaten mit den Soll-Koordinaten übereinstimmen würden.

Vorteilhafterweise können die Abweichungen zwischen den Ist-Koordinaten und den Soll-Koordinaten für verschiedene Abstände und Ausrichtungen der Referenzoberfläche relativ zur dentalen Kamera aus mehreren Aufnahmen ermittelt werden.

Die durch einen optischen Fehler verursachten Verzeichnungen können tiefenabhängig sein, so dass sich die Abweichungen zwischen den Ist-Koordinaten und den Soll-Koordinaten abhängig vom Abstand der Referenzoberfläche relativ zur dentalen Kamera verändern. Durch die Ermittlung der Kompensationswerte für verschiedene Abstände kann auch der tiefenabhängige optische Abbildungsfehler kompensiert werden.

Vorteilhafterweise kann für jede Kante eines Streifens jeweils nur ein Kompensationswert berechnet werden, der eine notwendige Verschiebung dieser Kante in der Ebene des Projektionsgitters angibt, um die Abweichungen für diese Kante zu kompensieren.

Bei dem Streifenprojektionsverfahren werden die beiden Kanten jedes Streifens ausgewertet, um die 3D-Koordinaten der Messpunkte entlang dieser Kanten zu berechnen. Der Kompensationswert für eine Kante kann beispielsweise berechnet werden, indem für alle Messpunkte der jeweiligen Kante Abweichungen zwischen den Ist-Koordinaten und den Soll-Koordinaten ermittelt werden und anschließend ein Mittelwert aus diesen Abweichungen gebildet wird. Ausgehend von diesem Mittelwert der Abweichungen wird dann der entsprechende Kompensationswert berechnet.

Vorteilhafterweise kann jede Kante eines Streifens in mehrere Sektoren unterteilt werden, wobei für jeden Sektor ein Kompensationswert berechnet wird, der eine notwendige Verschiebung dieses Sektors in der Ebene des Projektionsgitters angibt, um die Abweichungen für diesen Sektor zu kompensieren.

Dadurch wird jede Kante in mehrere Sektoren unterteilt, um die Kompensation zu verbessern. Denn bei einer gewellten Kante reicht es nicht aus, die gesamte Kante zu verschieben. Stattdessen wird für jeden der Sektoren ein eigener Kompensationswert berechnet, um den Fehler für diesen Sektor zu kompensieren.

Vorteilhafterweise können die Abweichungen zwischen den Ist-Koordinaten und den Soll-Koordinaten ermittelt werden, indem mehrere dreidimensionale Aufnahmen der Referenzoberfläche in der gleichen Lage und Ausrichtung relativ zur dentalen Kamera durchgeführt werden, wobei die Abweichungen aus den einzelnen Aufnahmen gemittelt werden, um einen durch ein Rauschsignal verursachten, nicht systematischen Fehler zu vermindern.

Dadurch werden durch das Rauschsignal verursachte Fehler herausgemittelt, so dass nur noch der systematische Fehler übrig bleibt, der beispielsweise durch einen Herstellungsfehler des Projektionsgitters oder durch Abbildungsfehler des optischen Systems verursacht wird.

Ein weiterer Gegenstand der Erfindung ist eine Kalibrierungsvorrichtung zur Kalibrierung einer dentalen Kamera, die auf einem Streifenprojektionsverfahren zur dreidimensionalen optischen Vermessung eines dentalen Objekts beruht, wobei die dentale Kamera ein Projektionsgitter zur Erzeugung eines Projektionsmusters aus mehreren Streifen und ein optisches System, das das erzeugte Projektionsmuster auf das zu vermessende Objekt projiziert, umfasst. Die Kalibrierungsvorrichtung weist eine erste Haltevorrichtung für die dentale Kamera und eine zweite Haltevorrichtung für eine Referenzoberfläche auf, wobei die erste Haltevorrichtung relativ zur zweiten Haltevorrichtung so verstellbar ist, dass mehrere definierte Abstände und/oder Ausrichtungen zwischen der dentalen Kamera und der Referenzoberfläche einstellbar sind.

Dadurch kann das oben erläuterte Verfahren zur Kalibrierung der dentalen Kamera durchgeführt werden. Mittels dieser Kalibrierungsvorrichtung können mehrere Aufnahmen für verschiedene Abstände und/oder Ausrichtungen zwischen der dentalen Kamera und der Referenzoberfläche durchgeführt werden. Anschließend kann für jede der Aufnahmen eine Matrix aus Kornpensationswerten berechnet werden, die zur Korrektur des Projektionsmusters verwendet wird. Auf diese Weise können durch eine fehlerhafte Optik verursachte abstandsabhängige Abbildungsfehler kompensiert werden. Vorteilhafterweise kann die erste Haltevorrichtung ein Innengewinde und die zweite Haltevorrichtung ein Außengewinde aufweisen, das in das Innengewinde der ersten Haltevorrichtung greift. Durch die Drehung der ersten Haltevorrichtung relativ zur zweiten Haltevorrichtung sind dadurch der Abstand und die Ausrichtung der dentalen Kamera relativ zur Referenzoberfläche in definierten Schritten veränderbar. Durch eine solche Gewindeverbindung können der Abstand und die Ausrichtung der Referenzoberfläche relativ zur Kamera auf eine einfache Art und Weise durch die Drehung schrittweise verändert werden.

Vorteilhafterweise kann die ebene Referenzoberfläche mehrere quadratische Markierungen aufweisen, die in bekannten Abständen zueinander angeordnet sind.

Dadurch kann anhand der Markierungen die genaue Lage der Referenzoberfläche relativ zur Kamera bestimmt werden. Dazu können bekannte Kalibrierverfahren verwendet werden.

### Kurzbeschreibung der Zeichnungen

Die Erfindung wird anhand der Zeichnungen erläutert. Es zeigt, die
- Fig. 1: eine Skizze einer dentalen Kamera, die auf einem Streifenprojektionsverfahren beruht; die
- Fig. 2: eine Skizze eines verzerrten Projektionsgitters aus Fig. 1; die
- Fig. 3: eine Skizze eines verzerrten Projektionsmusters; die
- Fig. 4: eine Skizze eines Kameramodell der dentalen Kamera aus Fig. 1; die
- Fig. 5: eine Skizze zur Verdeutlichung eines Kompensationsverfahrensschritts; die
- Fig. 6: eine Skizze zur Verdeutlichung einer weiteren Ausführungsform des Kompensationsverfahrensschritts; die
- Fig. 7: eine Skizze einer Kalibrierungsvorrichtung; die
- Fig. 8: eine Kalibrierungsplatte mit Markierungen.

### Ausführungsbeispiel

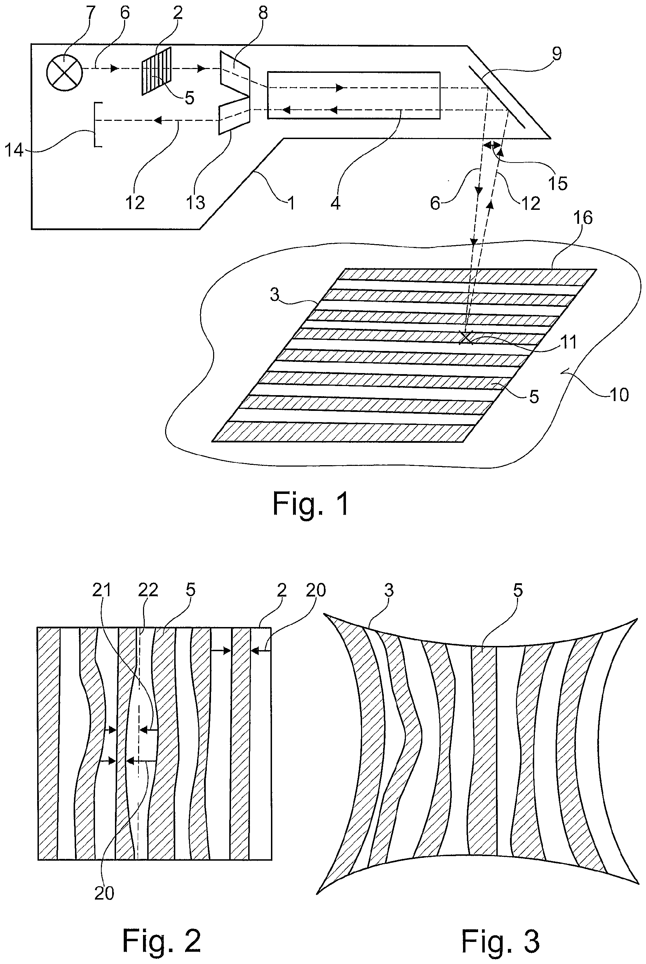

Die Fig. 1 zeigt eine Skizze einer dentalen Kamera 1, die auf einem Streifenprojektionsverfahren zur dreidimensionalen optischen Vermessung beruht. Die dentale Kamera 1 umfasst ein Projektionsgitter 2 zur Erzeugung eines Projektionsmusters 3 aus mehreren parallelen Streifen und ein optisches System 4, das das erzeugte Projektionsmuster 3 auf das nicht dargestellte zu vermessende Objekt projiziert. Das Projektionsgitter kann beispielsweise ein Dia mit mehreren parallelen Streifen 5 sein. Ein Beleuchtungsstrahl 6 wird von einer Lichtquelle 7, wie einer LED, erzeugt, durchstrahlt das Projektionsgitter 2, wird mittels eines Umlenkprismas 8 umgelenkt und anschließend mittels des optischen Systems 4 und eines Umlenkspiegels 9 auf das zu vermessende Objekt 10 projiziert. Das Objekt 10 kann beispielsweise die Oberfläche eines Zahns sein. Der Beleuchtungsstrahl 6 wird dann von einem Messpunkt 11, der als ein Kreuz dargestellt ist, als ein Beobachtungsstrahl 12 zurückgestrahlt. Der Beobachtungsstrahl 12 wird dann mittels des Umlenkspiegels 9, mittels des optischen Systems 4 und mittels eines zweiten Umlenkprismas 13 zu einem Bilddetektor 14 umgelenkt. Der Bilddetektor 14 kann beispielsweise ein CCD-Detektor oder ein CMOS-Detektor sein. Unter Verwendung des Streifenprojektionsverfahrens wird anhand eines Triangulationswinkels 15 zwischen dem Beleuchtungsstrahl 6 und dem Beobachtungsstrahl 12 sowie anhand der bekannten Positionen der Lichtquelle 7 und des Bilddetektors 14 eine 3D-Koordinate des Messpunkts 11 relativ zur dentalen Kamera 1 bestimmt. Auf diese Weise wird das zu vermessende Objekt 10 innerhalb eines Messfeldes 16 des projizierten Projektionsmusters 3 vollständig vermessen. Zur Identifikation der einzelnen Streifen 5 des Projektionsmusters 3 können die einzelnen Streifen unter Verwendung unterschiedlicher Lichteigenschaften, wie Intensität, Farbe, Polarisation, Kohärenz, Phase, Kontrast, Ort oder Laufzeit, kodiert sein.

Bei einer farblichen Kodierung kann der jeweilige Streifen eindeutig anhand der benachbarten Streifen identifiziert werden, wobei beispielsweise eine Reihenfolge von vier benachbarten Farbstreifen bzw. von drei Farbübergängen eindeutig kodiert sein kann.

Bei einer Kodierung mittels der Phase können die einzelnen Streifen längs zum Verlauf der Streifen 5 ein Sinusmuster aufweisen, wobei das Sinusmuster in seiner Phase für die einzelnen Streifen 5 leicht verschoben ist. Anhand der Phasenverschiebung kann dann der jeweilige Streifen eindeutig identifiziert werden.

Die Streifenbreite für solche Streifenprojektionsverfahren kann beispielsweise 150 µm im Messfeld 16 am zu vermessenden Objekt 10 betragen.

Herstellungsfehler des Projektionsgitters 2 sowie Abbildungsfehler der optischen Systems 4 können dazu führen, dass das Projektionsmuster 3 verzerrt abgebildet wird. Dies führt zu Messfehlern bei der Ermittlung der 3D-Koordinaten der Messpunkte auf der Oberfläche des Messobjekts 10. Zur Kompensation dieser Messfehler wird das vorliegende erfinderische Verfahren durchgeführt.

Die Fig. 2 zeigt eine Skizze des Projektionsgitters 2 aus Fig. 1 mit parallelen Streifen 5, die in ihrer Helligkeit variieren. Die dunklen Streifen sind gestrichelt dargestellt. Die Streifen 5 sind durch die Herstellungsfehler verzerrt, so dass eine Ist-Streifenbreite 20 der einzelne Streifen 5 von einer Soll-Streifenbreite 21 abweicht. Beispielhaft ist am dritten Streifen von links eine Soll-Streifenkante 22 gestrichelt dargestellt. Die Abweichung zwischen der Ist-Streifenbreite 20 und der Soll-Streifenbreite 21 führt zu Messfehlern der erzeugten dreidimensionalen Aufnahme.

Die Fig. 3 zeigt das auf das Objekt 10 aus Fig. 1 projizierte Projektionsmuster 3 mit Streifen 5, wobei die durch den Herstellungsfehler verzerrten Streifen aus Fig. 2 zusätzlich durch den Abbildungsfehler des optischen Systems 4 aus Fig. 1 verzerrt werden. Die sogenannten optischen Verzeichnungen können beliebig gestaltet sein. Im vorliegenden Fall handelt es sich um eine kissenförmige Verzeichnung, bei der die Ecken des Projektionsmusters 3 auseinandergedehnt werden. Bei der Verzeichnung könnte es sich auch um eine rotationssymmetrische tonnenförmige Verzeichnung handeln, wobei die Ecken des Projektionsmusters zur Mitte hin verzerrt werden. Diese optischen Verzeichnungen führen also ebenfalls zu einem Messfehler der dreidimensionalen Aufnahme des Objekts 10.

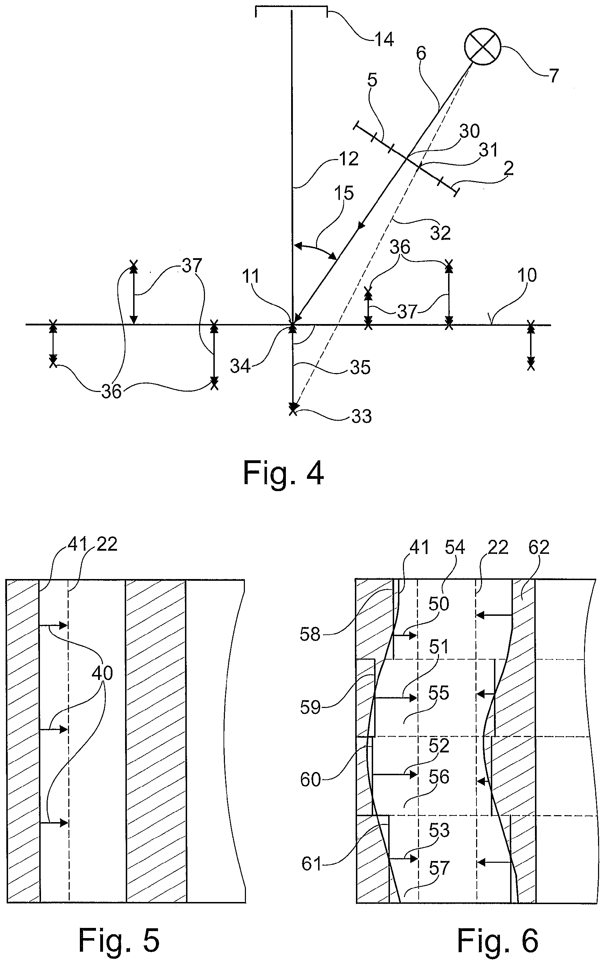

Die Fig. 4 zeigt eine Skizze zur Verdeutlichung des vorliegenden Verfahrens, wobei ein Kameramodell der dentalen Kamera aus Fig. 1 dargestellt ist. Die Lichtquelle 7 sendet den Beleuchtungsstrahl 6 aus. Das Projektionsgitter 2 weist mehrere parallele Streifen 5 auf, die durch die kurzen Striche angedeutet sind. Das Projektionsmuster wird auf die Oberfläche des Messobjekts 10 projiziert. Dabei ist skizzenhaft dargestellt, dass der Beleuchtungsstrahl 6 an einem Messpunkt 11 reflektiert wird und als ein Beobachtungsstrahl 12 zum Bilddetektor 14 zurückgestrahlt wird. Anhand des Triangulationswinkels 15 sowie der Positionen der Lichtquelle 7 und des Bilddetektors 14 wird dann die 3D-Koordinate des Messpunkts 11 relativ zur dentalen Kamera 1 berechnet. Durch die in Fig. 2 und Fig. 3 verursachten Verzerrungen des Projektionsmusters 3 ist eine gewünschte Soll-Streifenkante 30 zu der tatsächlichen fehlerhaften Ist-Sreifenkante 31 verschoben. Dadurch ist also der Beleuchtungsstrahl 6 ebenfalls zu einem veränderten Beleuchtungsstrahl 32, der gestrichelt dargestellt ist, verschoben. Der verschobene fehlerhafte Beleuchtungsstrahl 32 kreuzt den Beobachtungsstrahl 12 also bei einer Ist-Koordinate 33 des Messpunkts 11. Diese Ist-Koordinate 33 weist erheblich von einer Soll-Koordinate 34 des Messpunkts 11 ab. Zur Durchführung der Kalibrierung ist das aufzunehmende Objekt 10 eine ebene Referenzoberfläche. Zur Kalibrierung wird also eine Abweichung 35 zwischen der Ist-Koordinate 33 und der Soll-Koordinate 34 des Messpunkts 11 ermittelt. Die Abweichung 35 kann beispielsweise senkrecht zur Referenzebene 10 bestimmt werden. Auf diese Weise werden die Ist-Koordinaten 36 und die Abweichungen 37 zu den übrigen Messpunkten auf der Referenzebene 10 ermittelt. Zur Bestimmung der genauen Lage der Referenzebene 10 relativ zur dentalen Kamera 1 kann die Referenzebene Markierungen mit bekannten Abständen zueinander aufweisen. Anhand der Markierungen können also die genaue Lage der Referenzebene 10 relativ zur dentalen Kamera 1 und anschließend die Abweichungen 35, 37 bestimmt werden.

Alternativ dazu kann durch die ermittelten Ist-Koordinaten 33, 36 eine Bezugsebene gefittet werden, wobei anschließend die Abweichungen zwischen den Ist-Koordinaten 33, 36 und der Bezugsebene bestimmt werden. Ausgehend von den Abweichungen 35, 37 werden dann Kompensationswerte für die einzelnen Streifen 5 ermittelt, die diese Abweichungen kompensieren.

Die Fig. 5 zeigt eine Skizze zur Verdeutlichung des Kompensationsverfahrensschritts. Dabei wird ausgehend von den Abweichungen 35, 37 ein Kompensationswert 40 berechnet, der eine notwendige Verschiebung einer Ist-Streifenkante 41 zu der gewünschten Soll-Streifenkante 22 angibt, um die Abweichungen 35, 37 für die Ist-Streifenkante 41 zu kompensieren. Auf diese Weise erfolgt die Kompensation für alle übrigen Streifen 5 aus Fig. 3.

Die Fig. 6 zeigt eine Skizze für eine weitere Ausführungsform des Kompensationsverfahrensschritts. Im Vergleich zu Fig. 5 wird für die Ist-Streifenkante 41 nicht nur ein Kompensationswert, sondern mehrere Kompensationswerte 50, 51, 52 und 53 für vier Sektoren 54, 55, 56 und 57 ermittelt. Dazu werden die Mittelwerte 58, 59, 60 und 61 für die einzelnen Sektoren 54, 55, 56 und 57 der Ist-Streifenkante und anschließend die Abweichungen 50, 51, 52 und 53 zu der Soll-Streifenkante 22 bestimmt. Auf diese Weise werden die Kompensationswerte für die unterschiedlichen Sektoren 54, 55, 56 und 57 auch für die übrigen Streifen 5 bestimmt, wie anhand einer zweiten Ist-Streifenkante 62 dargestellt ist. Es wird also eine Matrix aus Kompensationswerten berechnet, die in einem Kompensationsverfahrensschritt während der Vermessung des Objekts 10 auf das Projektionsmuster 3 angewendet wird, um das Projektionsmuster 3 zu korrigieren. Anschließend wird unter Verwendung des korrigierten Projektionsmusters die dreidimensionale Aufnahme des Objekts 10 berechnet.

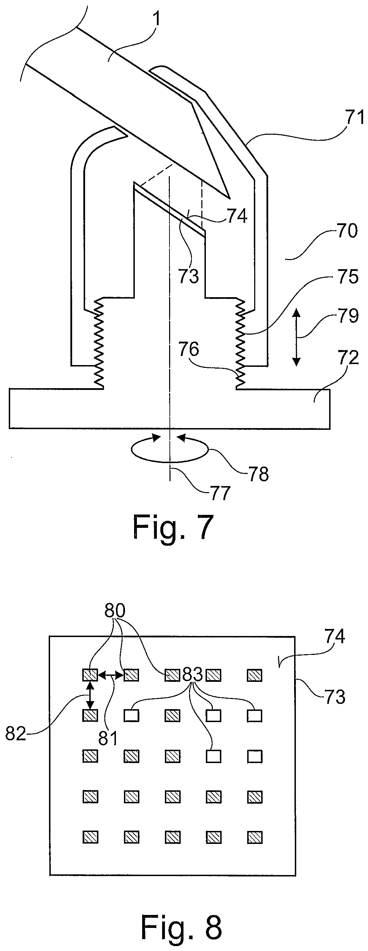

Die Fig. 7 zeigt eine Skizze einer Kalibrierungsvorrichtung 70, die eine erste Haltevorrichtung 71 für die dentale Kamera 1 aus Fig. 1 und eine zweite Haltevorrichtung 72 für eine Kalibrierungsplatte 73 mit einer Referenzoberfläche 74 aufweist. Die erste Haltevorrichtung 71 weist ein Innengewinde 75 und die zweite Haltevorrichtung 72 ein Außengewinde 76 auf, die ineinander greifen. Dadurch werden durch eine Drehung der zweiten Haltevorrichtung 72 relativ zur ersten Haltevorrichtung 71 um eine Drehachse 77, wie durch den Pfeil 78 dargestellt ist, die Lage und Orientierung zwischen der dentalen Kamera 1 und der Referenzoberfläche 74 auf eine definierte Weise verändert, wie durch den Pfeil 79 dargestellt ist. Das Innengewinde 75 und das Außengewinde 76 können Einrastvorrichtungen aufweisen, um die Drehung 78 in definierten Schritten zu gewährleisten. Dadurch können mehrere Aufnahmen der Referenzoberfläche 74 wiederholbar in definierten Abständen und mit definierten Winkeln zwischen der Dentalkamera 1 und der Referenzoberfläche 74 durchgeführt werden. Das vorliegenden Kalibrierungsverfahren kann also für verschiedene Abstände und Winkel durchgeführt werden, so dass für die verschiedenen Abstände und Winkel jeweils eine Matrix aus Kompensationswerten 50, 51, 52 und 53 aus Fig. 6 ermittelt wird. Auf diese Weise können durch optische Verzeichnungen verursachte tiefenabhängige Messfehler kompensiert werden.

Die Fig. 8 zeigt die Kalibrierungsplatte 73 mit der Referenzoberfläche 74 aus Fig. 7, wobei die Referenzoberfläche 74 mit Markierungen 80 versehen ist, die definierte Abstände 81, 82 zueinander aufweisen. Die Markierungen können auch unterschiedliche Helligkeiten aufweisen. Mittels eines charakteristischen Musters 83 aus hellen Markierungen kann die genaue Lage der jeweiligen Aufnahme auf der Referenzoberfläche 74 ermittelt werden. Damit kann die eingestellte Lage und Ausrichtung der Referenzoberfläche relativ zur dentalen Kamera 1 überprüft werden.

### Bezugszeichen

- 1: dentale Kamera
- 2: Projektionsgitter
- 3: Projektionsmuster
- 4: optisches System
- 5: parallele Streifen
- 6: Beleuchtungsstrahl
- 7: Lichtquelle
- 8: Umlenkprisma
- 9: Umlenkspiegel
- 10: Messobjekt
- 11: Messpunkt
- 12: Beobachungsstrahl
- 13: Umlenkprisma
- 14: Bilddetektor
- 15: Triangulationswinkel
- 16: Messfeld
- 20: Ist-Streifenbreite
- 21: Soll-Streifenbreite
- 30: Soll-Streifenkante
- 31: Ist-Streifenkante
- 32: Beleuchtungsstrahl
- 33: Ist-Koordinate
- 34: Soll-Koordinate
- 35: Abweichung
- 36: Ist-Koordinaten
- 37: Abweichungen
- 40: Kompensationswert
- 41: Ist-Streifenkante
- 50: Kompensationswert
- 51: Kompensationswert
- 52: Kompensationswert
- 53: Kompensationswert
- 54: erster Sektor
- 55: zweiter Sektor
- 56: dritter Sektor
- 57: vierter Sektor
- 58: erster Mittelwert
- 59: zweiter Mittelwert
- 60: dritter Mittelwert
- 61: vierter Mittelwert
- 70: Kalibrierungsvorrichtung
- 71: erste Haltevorrichtung
- 72: zweite Haltevorrichtung
- 73: Kalibrierungsplatte
- 74: Referenzoberfläche
- 75: Innengewinde
- 76: Außengewinde
- 77: Drehachse
- 78: Drehbewegung
- 79: Verstellung
- 80: Markierungen
- 81: erster Abstand
- 82: zweiter Abstand
- 83: Muster aus hellen Markierungen

## Patentansprüche

1. Verfahren zur Kalibrierung einer dentalen Kamera (1), die auf einem Streifenprojektionsverfahren zur dreidimensionalen optischen Vermessung eines dentalen Objekts (10) beruht, umfassend ein Projektionsgitter (2) zur Erzeugung eines Projektionsmusters (3) aus mehreren Streifen (5) und ein optisches System (4), das das erzeugte Projektionsmuster (3) auf das zu vermessende Objekt (10) projiziert, wobei mittels der dentalen Kamera (1) eine Referenzoberfläche (74) mit bekannten Abmessungen unter Verwendung des Streifenprojektionsverfahrens vermessen wird und dabei Ist-Koordinaten (33, 36) von mehreren Messpunkten (11) auf der Referenzoberfläche (74) ermittelt werden, wobei die ermittelten Ist-Koordinaten (33, 36) mit bekannten Soll-Koordinaten (34) der Messpunkte (11) auf der Referenzoberfläche (74) mit bekannten Abmessungen verglichen werden, wobei ausgehend von den Abweichungen (35, 37) zwischen den Ist-Koordinaten (33, 36) und den Soll-Koordinaten (34) mehrere Kompensationswerte (40, 50, 51, 52, 53) für die einzelnen Streifen (5) des Projektionsmusters (3) berechnet werden, wobei während der Vermessung des dentalen Objekts (10) die berechneten Kompensationswerte (40, 50, 51, 52, 53) berücksichtigt werden, um die Abweichungen (35, 37) zwischen den Ist-Koordinaten (33, 36) und den Soll-Koordinaten (34) zu kompensieren, wobei die Kalibrierung mittels einer Kalibrierungsvorrichtung erfolgt, die eine erste Haltevorrichtung (71) für die dentale Kamera (1) und eine zweite Haltevorrichtung (72) für eine Referenzoberfläche (74) aufweist, wobei die erste Haltevorrichtung (71) relativ zur zweiten Haltevorrichtung (72) so verstellbar ist, dass mehrere definierte Abstände und/oder Ausrichtungen zwischen der dentalen Kamera (1) und der Referenzoberfläche (74) einstellbar sind, wobei durch die Drehung der ersten Haltevorrichtung (71) relativ zur zweiten Haltevorrichtung (72) mittels eines Gewindes (76) der Abstand und die Ausrichtung der dentalen Kamera (1) relativ zur Referenzoberfläche (74) in definierten Schritten verändert wird, wobei die ebene Referenzoberfläche (74) an der zweiten Haltevorrichtung (72) in einem Winkel zwischen 50° und 70° relativ zu einer Mittelachse (77) des Gewindes (76) angeordnet ist.

2. Verfahren nach Anspruch 1, **dadurch gekennzeichnet, dass** die Referenzoberfläche (74) eine Ebene ist und die Abweichungen (35, 37) zwischen den Ist-Koordinaten (33, 36) und den Soll-Koordinaten (34) ermittelt werden, indem durch die Ist-Koordinaten (33, 36) unter Verwendung der Methode der kleinsten Quadrate eine Bezugsebene gefittet wird und anschließend die Abweichungen (35, 37) zwischen den Ist-Koordinaten (33, 36) und der Bezugsebene entlang einer Richtung senkrecht zur Bezugsebene ermittelt werden.

3. Verfahren nach Anspruch 1 oder 2, **dadurch gekennzeichnet, dass** die Abweichungen (35, 37) zwischen den Ist-Koordinaten (33, 36) und den Soll-Koordinaten (34) für verschiedene Abständen und Ausrichtungen der Referenzoberfläche (74) relativ zur dentalen Kamera (1) aus mehreren Aufnahmen ermittelt werden.

4. Verfahren nach Anspruch 3, **dadurch gekennzeichnet, dass** die Aufnahmen mittels einer Kalibrierungsvorrichtung (70) durchgeführt werden, die eine erste Haltevorrichtung (71) für die dentale Kamera (1) und eine zweite Haltevorrichtung (72) für die Referenzoberfläche (74) aufweist, die auf eine definierte Weise in mehreren Schritten relativ zur ersten Haltevorrichtung (71) verstellbar ist.

5. Verfahren nach einem der Ansprüche 1 bis 4, **dadurch gekennzeichnet, dass** für jede Kante eines Streifens (5) jeweils nur ein Kompensationswert (40) berechnet wird, der eine notwendige Verschiebung dieser Kante (41) in der Ebene des Projektionsgitters (2) angibt, um die Abweichungen (35, 37) für diese Kante (41) zu kompensieren.

6. Verfahren nach einem der Ansprüche 1 bis 4, **dadurch gekennzeichnet, dass** jede Kante eines Streifens (5) in mehrere Sektoren (54, 55, 56, 57) unterteilt wird, wobei für jeden Sektor (54, 55, 56, 57) ein Kompensationswert (50, 51, 52, 53) berechnet wird, der eine notwendige Verschiebung dieses Sektors (54, 55, 56, 57) in der Ebene des Projektionsgitters (2) angibt, um die Abweichungen (35, 37) für diesen Sektor (54, 55, 56, 57) zu kompensieren.

7. Verfahren nach einem der Ansprüche 1 bis 6, **dadurch gekennzeichnet, dass** die Abweichungen (35, 37) zwischen den Ist-Koordinaten (33, 36) und den Soll-Koordinaten (34) ermittelt werden, indem mehrere dreidimensionale Aufnahmen der Referenzoberfläche (74) in der gleichen Lage und Ausrichtung relativ zur dentalen Kamera (1) durchgeführt werden, wobei die Abweichungen (35, 37) aus den einzelnen Aufnahmen gemittelt werden, um einen durch ein Rauschsignal verursachten, nicht systematischen Fehler zu vermindern.

8. Kalibrierungsvorrichtung zur Kalibrierung einer dentalen Kamera (1), die auf einem Streifenprojektionsverfahren zur dreidimensionalen optischen Vermessung eines dentalen Objekts (10) beruht, wobei die dentale Kamera ein Projektionsgitter (2) zur Erzeugung eines Projektionsmusters (3) aus mehreren Streifen (5) und ein optisches System (4), das das erzeugte Projektionsmuster (3) auf das zu vermessende Objekt (10) projiziert, umfasst, **dadurch gekennzeichnet, dass** die Kalibrierungsvorrichtung eine erste Haltevorrichtung (71) für die dentale Kamera (1) und eine zweite Haltevorrichtung (72) für eine Referenzoberfläche (74) aufweist, wobei die erste Haltevorrichtung (71) relativ zur zweiten Haltevorrichtung (72) so verstellbar ist, dass mehrere definierte Abstände und/oder Ausrichtungen zwischen der dentalen Kamera (1) und der Referenzoberfläche (74) einstellbar sind, wobei die erste Haltevorrichtung (71) ein Innengewinde (75) und die zweite Haltevorrichtung (72) ein Außengewinde (76) aufweist, das in das Innengewinde (75) der ersten Haltevorrichtung (71) greift, wobei durch die Drehung der ersten Haltevorrichtung (71) relativ zur zweiten Haltevorrichtung (72) der Abstand und die Ausrichtung der dentalen Kamera (1) relativ zur Referenzoberfläche (74) in definierten Schritten veränderbar sind, wobei die ebene Referenzoberfläche (74) an der zweiten Haltevorrichtung (72) in einem Winkel zwischen 50° und 70° relativ zu einer Mittelachse (77) des Außengewindes (76) angeordnet ist.

9. Kalibrierungsvorrichtung nach Anspruch 8, **dadurch gekennzeichnet, dass** die ebene Referenzoberfläche (74) mehrere quadratische Markierungen (80) aufweist, die in bekannten Abständen zueinander angeordnet sind.

## Claims

1. A method for calibrating a dental camera (1), which method is based on a fringe projection method for the three-dimensional optical measurement of a dental object (10), comprising a projection grating (2) for generating a projection pattern (3) made up of several fringes (5), and an optical system (4) which projects the generated projection pattern (3) onto the object to be measured (10); wherein a reference surface (74) with known dimensions is measured by means of the dental camera (1) using the fringe projection method, and in the process actual coordinates (33, 36) of several measurement points (11) on the reference surface (74) are determined; wherein the determined actual coordinates (33, 36) are compared to known target coordinates (34) of the measurement points (11) on the reference surface (74) with known dimensions; wherein - proceeding from the deviations (35, 37) between the actual coordinates (33, 36) and the target coordinates (34) - several compensation values (40, 50, 51, 52, 53) are calculated for the individual fringes (5) of the projection pattern (3); wherein during the measurement of the dental object (10) the calculated compensation values (40, 50, 51, 52, 53) are taken into account in order to compensate for the deviations (35, 37) between the actual coordinates (33, 36) and the target coordinates (34); wherein the calibration is carried out by means of a calibration device which comprises a first holding device (71) for the dental camera (1) and a second holding device (72) for a reference surface (74); wherein the first holding device (71) can be adjusted relative to the second holding device (72) such that several defined distances and/or alignments between the dental camera (1) and the reference surface (74) can be set; wherein the distance and the alignment of the dental camera (1) relative to the reference surface (72) is changed in defined steps via the rotation of the first holding device (71) relative to the second holding device (72) by means of a thread (76); wherein the flat reference surface (74) is arranged on the second holding device (72) at an angle between 50° and 70° relative to a center axis (77) of the thread (76).

2. The method according to claim 1, **characterized in that** the reference surface (74) is a plane and the deviations (35, 37) between the actual coordinates (33, 36) and the target coordinates (34) are determined by fitting a reference plane by means of the actual coordinates (33, 36) using the least squares method, and subsequently determining the deviations (35, 37) between the actual coordinates (33, 36) and the reference plane along a direction orthogonal to the reference plane.

3. The method according to claim 1 or 2, **characterized in that** the deviations (35, 37) between the actual coordinates (33, 36) and the target coordinates (34) are determined from several exposures for different distances and alignments of the reference surface (74) relative to the dental camera (1).

4. The method according to claim 3, **characterized in that** the exposures are taken by means of a calibration device (70) which comprises a first holding device (71) for the dental camera (1) and a second holding device (72) for the reference surface (74), which can be adjusted in a defined manner in several steps relative to the first holding device (71).

5. The method according to one of claims 1 to 4, **characterized in that**, for each edge of a fringe (5), respectively only one compensation value (40) is calculated which indicates a necessary displacement of this edge (41) in the plane of the projection grating (2) in order to compensate for the deviations (35, 37) for this edge (41).

6. The method according to one of claims 1 to 4, **characterized in that** each edge of a fringe (5) is subdivided into several sectors (54, 55, 56, 57), wherein for each sector (54, 55, 56, 57) a compensation value (50, 51, 52, 53) is calculated which indicates a necessary displacement of this sector (54, 55, 56, 57) in the plane of the projection grating (2) in order to compensate for the deviations (35, 37) for this sector (54, 55, 56, 57).

7. The method according to one of claims 1 to 6, **characterized in that** the deviations (35, 37) between the actual coordinates (33, 36) and the target coordinates (34) are determined by taking several three-dimensional exposures of the reference surface (74) in the same position and alignment relative to the dental camera (1), wherein the deviations (35, 37) are determined from the individual exposures in order to reduce a non-systematic error caused by a noise signal.

8. A calibration device for calibrating a dental camera (1), which calibration device is based on a fringe projection method for the three-dimensional optical measurement of a dental object (10), wherein the dental camera comprises a projection grating (2) for generating a projection pattern (3) made up of several fringes (5) and an optical system (4) which projects the generated projection pattern (3) onto the object to be measured (10), **characterized in that** the calibration device comprises a first holding device (71) for the dental camera (1) and a second holding device (72) for a reference surface (74), wherein the first holding device (71) can be adjusted relative to the second holding device (72) such that several defined distances and/or alignments between the dental camera (1) and the reference surface (74) can be set; wherein the first holding device (71) comprises an inner thread (75) and the second holding device (72) comprises an outer thread (76) which engages with the inner thread (75) of the first holding device (71); wherein the distance and alignment of the dental camera (1) relative to the reference surface (74) can be changed in defined steps by the rotation of the first holding device (71) relative to the second holding device (72); wherein the flat reference surface (74) is arranged on the second holding device (72) at an angle between 50° and 70° relative to a center axis (77) of the outer thread (76).

9. The calibration device according to claim 8, **characterized in that** the flat reference surface (74) comprises several square markings (80) which are arranged at known distances to each other.

## Revendications

1. Procédé d'étalonnage d'une caméra dentaire (1), reposant sur un procédé de projection de franges pour la mesure optique tridimensionnelle d'un objet dentaire (10), comprenant une grille de projection (2) pour la création d'un motif de projection (3) à partir de plusieurs franges (5) et un système optique (4), projetant le motif de projection créé (3) sur l'objet à mesurer (10), où, au moyen de la caméra dentaire (1), une surface de référence (74) dont les dimensions sont connues est mesurée en utilisant le procédé de projection de franges et ce faisant, les coordonnées réelles (33, 36) de plusieurs points de mesure (11) sur la surface de référence (74) sont déterminées, où les coordonnées réelles (33, 36) déterminées sont comparées aux coordonnées théoriques (34) connues des points de mesure (11) sur la surface de référence (74) dont les dimensions sont connues, où, à partir des écarts (35, 37) entre les coordonnées réelles (33, 36) et les coordonnées théoriques (34), plusieurs valeurs de compensation (40, 50, 51, 52, 53) sont calculées pour les franges individuelles (5) du motif de projection (3), où, lors de la mesure de l'objet dentaire (10, les valeurs de compensation (40, 50, 51, 52, 53) sont prises en compte, afin de compenser les écarts (35, 37) entre les coordonnées réelles (33, 36) et les coordonnées théoriques (34), où l'étalonnage s'effectue au moyen d'un dispositif d'étalonnage, qui présente un premier dispositif de support (71) pour la caméra dentaire (1) et un second dispositif de support (72) pour une surface de référence, où le premier dispositif de support (71) peut être réglé par rapport au second dispositif de support (72), de telle sorte que plusieurs distances et/ou orientations définies entre la caméra dentaire (1) et la surface de référence (74) soient réglables, où, la rotation du premier dispositif de support (71) par rapport au second dispositif de support (72) au moyen d'un filetage (76), permet de modifier la distance et l'orientation de la caméra dentaire (1) par rapport à la surface de référence (74), par incréments fixes, où la surface de référence (74) plane est agencée sur le second dispositif de support (72) selon un angle compris entre 50 ° et 70 ° par rapport à un axe médian (77) du filetage (76).

2. Procédé selon la revendication 1, **caractérisé en ce que** la surface de référence (74) représente un plan et les écarts (35, 37) entre les coordonnées réelles (33, 36) et les coordonnées théoriques (34) sont déterminés en adaptant un plan de référence au moyen des coordonnées réelles (33, 36) en utilisant la méthode des moindres carrés, puis les écarts (35, 37) entre les coordonnées réelles (33, 36) et le plan de référence sont déterminés suivant une direction perpendiculaire au plan de référence.

3. Procédé selon la revendication 1 ou 2, **caractérisé en ce que** les écarts (35, 37) entre les coordonnées réelles (33, 36) et les coordonnées théoriques (34) sont déterminés pour plusieurs distances et orientations de la surface de référence (74) par rapport à la caméra dentaire (1) à partir de plusieurs enregistrements.

4. Procédé selon la revendication 3, **caractérisé en ce que** les enregistrements sont réalisés au moyen d'un dispositif d'étalonnage (70), qui présente un premier dispositif de support (71) pour la caméra dentaire (1) et un second dispositif de support (72) pour la surface de référence (74), réglable d'une manière définie selon plusieurs incréments par rapport au premier dispositif de support (71).

5. Procédé selon l'une des revendications 1 à 4, **caractérisé en ce que**, pour chaque bord d'une frange (5), une seule valeur de compensation (40) est calculée respectivement, indiquant un déplacement requis de ce bord (41) dans le plan de la grille de projection (2), afin de compenser les écarts (35, 37) pour ce bord (41).

6. Procédé selon une des revendications 1 à 4, **caractérisé en ce que** chaque bord d'une frange (5) est divisé en plusieurs secteurs (54, 55, 56, 57), où, pour chaque secteur (54, 55, 56, 57), une valeur de compensation (50, 51, 52, 53) est calculée, indiquant un déplacement requis de ce secteur (54, 55, 56, 57) dans le plan de la grille de projection (2), afin de compenser les écarts (35, 37) pour ce secteur (54, 55, 56, 57).

7. Procédé selon l'une des revendications 1 à 6, **caractérisé en ce que** les écarts (35, 37) entre les coordonnées réelles (33, 36) et les coordonnées théoriques (34) sont déterminés en réalisant plusieurs enregistrements tridimensionnels de la surface de référence (74) dans la même position et la même orientation par rapport à la caméra dentaire (1), où les écarts (35, 37) sont déterminés à partir des enregistrements individuels, afin d'éviter toute erreur non systématique provoquée par un signal de fumée.

8. Dispositif d'étalonnage pour l'étalonnage d'une caméra dentaire (1), reposant sur un procédé de projection de franges pour la mesure optique tridimensionnelle d'un objet dentaire (10), où la caméra dentaire comprend une grille de projection (2) pour la création d'un motif de projection (3) à partir de plusieurs franges (5) et un système optique (4), qui projette le motif de projection créé (3) sur l'objet à mesurer (10), **caractérisé en ce que** le dispositif d'étalonnage présente un premier dispositif de support (71) pour la caméra dentaire (1) et un second dispositif de support (72) pour une surface de référence (74), où le premier dispositif de support (71) peut être déplacé par rapport au second dispositif de support (72), de sorte que plusieurs distances et/ou orientations définies entre la caméra dentaire (1) et la surface de référence (74) soient réglables, où le premier dispositif de support (71) présente un filetage interne (75) et le second (72) un filetage externe (76), qui vient en prise dans le filetage interne (75) du premier dispositif de support (71), où la rotation du premier dispositif de support (71) par rapport au second (72), permet de modifier la distance et l'orientation de la caméra dentaire (1) par rapport à la surface de référence (74), par incréments fixes, où la surface de référence (74) plane est agencée sur le second dispositif de support (72) selon un angle compris entre 50 ° et 70 ° par rapport à un axe médian (77) du filetage

9. Dispositif d'étalonnage selon la revendication 8, **caractérisé en ce que** la surface de référence plane (74) présente plusieurs graduations quadratiques (80), agencées à des distances connues les unes des autres.
